Europäisches Patentamt

European Patent Office

Office européen des brevets

(19)

(11) **EP 0 659 271 B1**

(12) **FASCICULE DE BREVET EUROPEEN**

(45) Date de publication et mention
de la délivrance du brevet:
**09.07.1997 Bulletin 1997/28**

(21) Numéro de dépôt: **93919439.5**

(22) Date de dépôt: **10.09.1993**

(51) Int Cl.$^6$: **G01N 25/18**

(86) Numéro de dépôt international:
**PCT/FR93/00868**

**WO 94/05999 (17.03.1994 Gazette 1994/07)**

(54) **DISPOSITIF DE MESURE DE PARAMETRES TELS QUE LA CONDUCTIVITE OU LA CAPACITE CALORIFIQUE D'UN MATERIAU INJECTABLE OU NON ET PROCEDE D'IDENTIFICATION DESDITS PARAMETRES**

VORRICHTUNG ZUR MESSUNG VON PARAMETERN WIE WÄRMELEITFÄHIGKEIT ODER WÄRMEKAPAZITÄT EINES EINSPRITZBAREN ODER NICHT-EINSPRITZBAREN MATERIALS UND VERFAHREN ZUR IDENTIFIZIERUNG DIESER PARAMETER

DEVICE FOR MEASURING PARAMETERS SUCH AS THERMAL CONDUCTIVITY OR HEAT CAPACITY OF AN INJECTABLE OR NON-INJECTABLE MATERIAL AND METHOD OF IDENTIFYING SAID PARAMETERS

(84) Etats contractants désignés:
**BE DE ES FR GB IT PT**

(30) Priorité: **10.09.1992 FR 9210805**

(43) Date de publication de la demande:
**28.06.1995 Bulletin 1995/26**

(73) Titulaire: **UNIVERSITE DE NANTES
F-44035 Nantes Cédex 01 (FR)**

(72) Inventeurs:
• **JURKOWSKI, Thomasz
F-44300 Nantes (FR)**
• **JARNY, Yvon
F-44700 Orvault (FR)**
• **DELAUNAY, Didier
F-44300 Nantes (FR)**

(74) Mandataire: **Dawidowicz, Armand et al
Cabinet Dawidowicz,
18, Boulevard Péreire
75017 Paris (FR)**

(56) Documents cités:
**EP-A- 0 124 104**

• **JOURNAL OF APPLIED PHYSICS, vol.60, no.2, Juillet 1986, NEW YORK US pages 493 - 498 E. PIORKOWSKA ET AL. 'Measurements of thermal conductivity of materials using a transient technique. II. Description of the apparatus'**
• **REVUE DE PHYSIQUE APPLIQUEE, vol.21, no.3, Mars 1986, PARIS FR pages 229 - 237 A. DEGIOVANNI ET AL. 'Une nouvelle technique d'identification de la diffusivité thermique pour la méthode "flash"'**
• **POLYMER ENGINEERING AND SCIENCE, vol.30, no.2, Janvier 1990 H. LOBO ET AL. 'Measurement of Thermal Conductivity of Polymer Melts by the Line-Source Method' cité dans la demande**
• **REVUE GéNéRALE DE THERMIQUE, vol.XVI, no.185, Mai 1977 pages 420 - 442 A. DEGIOVANNI 'Diffusivité et méthode flash' cité dans la demande**

## Description

La présente invention concerne un dispositif de mesure pour mesurer en régime transitoire des paramètres tels que la conductivité ou la capacité calorifique d'un matériau injectable ou non et établir éventuellement les lois cinétiques dudit matériau.

Les méthodes consistant à déterminer les propriétés thermiques (conductivité ou capacité calorifique) de matériaux divers sont nombreuses. On connaît ainsi les méthodes dites de mesure en régime permanent qui présentent l'inconvénient d'être longues à mettre en oeuvre tout en ne permettant à chaque fois que la mesure d'une seule valeur. Ces méthodes telles que celles qui utilisent une plaque gardée chaude ou un flux radial ne permettent pas de visualiser l'évolution d'un matériau telle qu'un changement de structure au cours de la mesure.

Un autre grand groupe de méthodes de mesure concerne les méthodes de mesure en régime transitoire. Une première méthode dite méthode flash décrite notamment dans l'article de A. Degiovanni : "Diffusivité et méthode Flash", Revue Générale de Thermique, Tome XVI, N° 185 de mai 1977 présente l'inconvénient de ne fournir qu'une valeur moyenne pour un intervalle de variation donné de la température du matériau. Une seconde méthode mettant en oeuvre une sonde à choc décrite dans l'article A. lobo/C Cohen "Measurement of thermal conductivity of polymer melts by line-source methods" Polymer Engineering and Science - Janvier 1990, volume 30, N° 2 et étude du couplage transfert thermique et transformation physico-chimique rapport préliminaire 1987, B. GARNIER / D. DELAUNAY, présente une précision assez décevante (dans la fourchette 10 - 20 %). Cette méthode est en outre assez longue à mettre en oeuvre quand on souhaite faire une mesure à des températures élevées puisqu'il faut préalablement à la mesure porter conductivimètre et matériaux à une température homogène. En conséquence, les méthodes décrites ci-dessus sont difficilement utilisables si des transformations s'opèrent dans le matériau pendant la durée soit de préparation soit de réalisation de la mesure. Elles sont donc peu appliquées dans ce cas. Tel est le cas en particulier du procédé et du dispositif décrits dans le brevet EP-A-0.124.104. Dans ce brevet, il est proposé un dispositif similaire sur le plan physique au dispositif objet de l'invention pour déterminer la conductivité thermique et la capacité thermique de matières. La différence essentielle avec l'objet de l'invention réside dans l'utilisation d'un élément de chauffage disposé entre deux échantillons qui oblige à modifier linéairement la température et à supposer que dans une plage de température donnée la conductivité est constante. De ce fait, de nouveau, au moyen d'un tel dispositif, on ne tient pas compte des transformations éventuelles du matériau.

Un troisième groupe de méthode utilisant des algorithmes d'identification par méthode inverse permet d'éviter les défauts cités précédemment (durée de la manipulation, impossibilité de réaliser plus d'une mesure, impossibilité de fournir des résultats dans certains domaines de matériaux où s'opèrent des transformations du matériau). Leur précision cependant est parfois insuffisante. En effet, dans certaines configurations de mesure des flux, il est nécessaire de bien connaître la valeur de certaines résistances thermiques de contact ou la conductivité thermique de certains matériaux constitutifs du conductivimètre. Ces mesures supplémentaires ne sont pas toujours faciles à réaliser. En outre, ces méthodes nécessitent souvent l'implantation de capteurs à l'intérieur des échantillons posant alors un certain nombre de problèmes.

Le but de la présente invention est donc de proposer un dispositif de mesure de paramètres tels que la conductivité en régime transitoire qui permette ladite mesure au moyen d'une manipulation et d'une mise en oeuvre rapides inférieures à l'heure, fournissant des résultats dans des plages de températures où s'opèrent des transformations du matériau et en tenant compte des résistances de contact aux interfaces échantillon/éléments constitutifs du conductivimètre, de manière à s'affranchir d'une présence des capteurs à l'intérieur des échantillons.

Un autre but de la présente invention est de proposer un dispositif de mesure polyvalent susceptible d'identifier des paramètres tels que la conductivité ou la capacité de matériaux thermoplastiques ou composites en tenant compte des conditions réelles de fabrication industrielle desdits matériaux.

L'invention concerne à cet effet un dispositif de mesure tel que revendiqué dans la revendication 1.

Selon une forme de réalisation préférée de l'invention, le dispositif de mesure comprend un troisième capteur de température constitué par un thermocouple T3 intégré sur la paroi externe de la plaque du deuxième échangeur.

L'invention concerne encore un procédé d'identification de paramètres tels que la conductivité du matériau caractérisé en ce qu'on établit à partir du dispositif de mesure la courbe $T_2$ (t) appelée réponse objet, en ce qu'on réalise un modèle du type différences finies permettant d'obtenir une réponse modèle $\Theta$ (t), en ce qu'on définit un critère d'écart

$$J = \sum_{k=1}^{n} (\Theta k - T k)^2$$

$\Theta_k$: température calculée à l'instant k
$T_k$: température mesurée à l'instant k.

qui permet de minimiser l'écart entre les températures mesurées dans la plaque centrale et les températures calculées pour le même point et dans les mêmes instants pour le modèle, ladite minimisation présentant un caractère itératif de manière à ajuster successivement l'ensemble des paramètres selon une méthode de descente, en ce qu'on arrête la comparaison lorsque la valeur du critère d'écart répond à une condition d'arrêt, ladite condition d'arrêt étant réalisée lorsque $J = \Sigma$, $\Sigma$ étant considéré comme négligeable.

D'autres caractéristiques et avantages de l'invention apparaîtront encore à la lecture de la description qui suit et des dessins joints, lesquels description et dessins sont donnés surtout à titre d'exemples. Dans ces dessins :

la figure 1 représente une vue schématique simplifiée éclatée d'un conductivimètre conforme à l'invention ;
la figure 2 représente un schéma fonctionnel du procédé d'identification de la conductivité ;
la figure 3 représente une vue schématique simplifiée du conductivimètre et de la circulation de fluides dans les échangeurs ;
la figure 4 représente une vue de face d'un échangeur à plaque susceptible d'être utilisé dans le conductivimètre ;
la figure 5a représente la courbe de température mesurée par le thermocouple 1 en fonction du temps ;
la figure 5b représente la courbe de température mesurée par le thermocouple 2 en fonction du temps ;
la figure 6 représente la courbe du flux thermique calculé en fonction du temps ;
la figure 7 représente les courbes de températures de la plaque centrale mesurées et calculées après convergence de l'algorithme en fonction du temps ; et
la figure 8 représente une vue schématique simplifiée d'une presse incorporant le dispositif de mesure.

Le dispositif de mesure, objet de l'invention est destiné à caractériser et à mesurer la conductivité et/ou la capacité d'échantillons de matériaux, que lesdits matériaux aient été disposés au départ de la mesure ou soient injectés, lesdits matériaux pouvant être des matériaux composites, des polymères, des thermoplastiques, des matériaux de conductivité faible dont la conductivité serait par exemple inférieure à 2, etc.

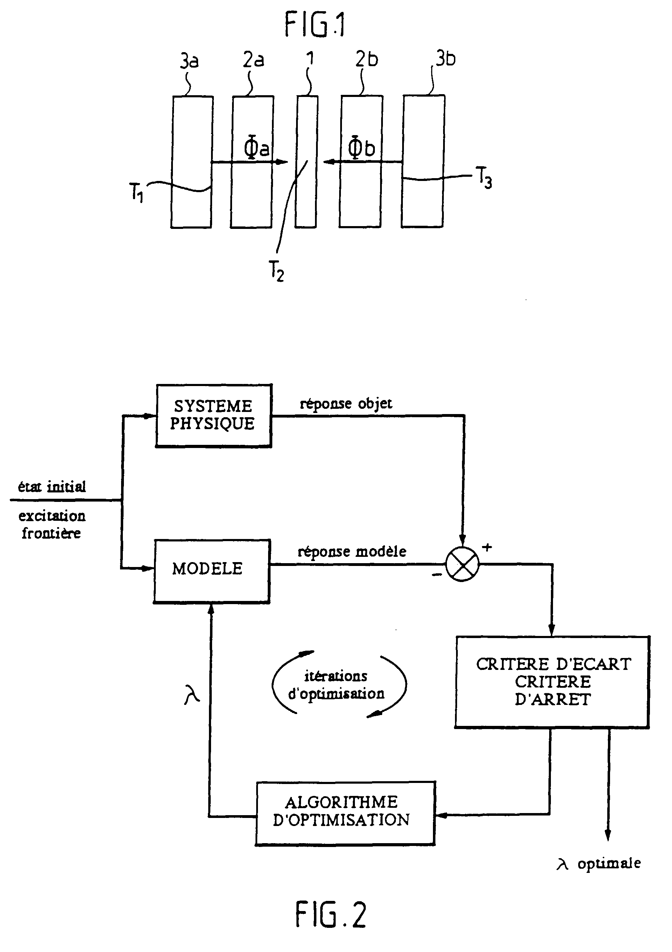

Les éléments constitutifs du dispositif de mesure en particulier dans le cas d'un conductivimètre comprennent, comme le montre la figure 1, deux échangeurs symétriques 3a, 3b à paroi plane permettant de réchauffer ou de refroidir un élément à leur contact et une plaque centrale 1 de séparation disposée entre lesdits échangeurs 3a et 3b de manière à ménager avec ces derniers deux chambres identiques. ces chambres sont délimitées d'une part, latéralement par les faces externes des échangeurs 3a et 3b décrits précédemment et par la plaque unique 1 de séparation, d'autre part, par une isolation qui sera définie ci-après et qui ferme les bords restant libres desdites chambres. A l'intérieur de ces chambres sont disposés les échantillons des matériaux à caractériser soit au départ de la manipulation soit au cours de la manipulation dans le cas où il s'agit d'un matériau injectable.

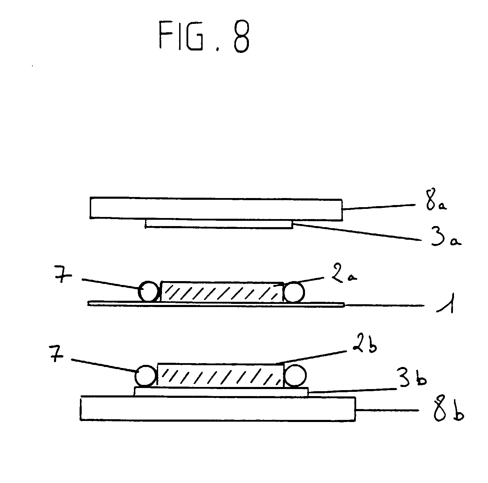

Associé à ces deux échangeurs de chaleur 3a et 3b et à cette plaque centrale 1 de séparation, un dispositif d'écartement et de serrage non représenté permet de positionner et de maintenir les échangeurs en contact de pression contre les échantillons eux-mêmes en contact de pression contre la plaque centrale de séparation. Dans le cas d'un matériau injectable, ce dispositif permet le maintien des échangeurs à distance connue de la plaque de séparation de telle sorte que les chambres soient de volume connu et puissent être remplies par exemple par injection à tout moment par un échantillon du matériau à caractériser. Dans le cas de matériaux composites, par exemple, il est possible de fixer respectivement les échangeurs 3a et 3b sur les faces en regard des plateaux mobiles 8a, 8b d'une presse puis de disposer les échantillons de matériaux composites 2a, 2b respectivement sur la plaque centrale 1 et sur l'échangeur 3b comme le montre la figure 8. Ces matériaux composites se présentant sous forme de strates imprégnées de résine sont maintenus au moyen d'un isolant 7. Lors du rapprochement des plateaux de la presse en direction de la plaque centrale 1 maintenue fixe, ces plateaux viennent comprimer au niveau de la face externe de l'échangeur l'échantillon de manière à assurer un contact de pression homogène entre les parois échangeurs/faces externes des échantillons/ faces de la plaque centrale. Bien évidemment, grâce à des capteurs, la distance entre les plateaux de la presse et la plaque centrale 1 est connue et prédéterminée de manière à permettre une analyse des paramètres identique à celle réalisée dans le cas où le dispositif de mesure n'est pas intégré dans une presse. De même, dans le cas de matières thermoplastiques injectables, il est possible de prévoir des buses d'injection débouchant dans chaque chambre généralement dans un plan radial au niveau de l'isolant 7 représenté à la figure 8. Grâce à de telles configurations, on reproduit les caractéristiques de fabrication industrielles et on peut tester le comportement d'un grand nombre de matériaux.

Pour obtenir un flux unidirectionnel au travers des deux échantillons du matériau à caractériser, il est nécessaire d'isoler l'ensemble du dispositif en fermant parfaitement le volume délimité par les parois respectives des échangeurs et de la plaque centrale 1. Cette isolation est obtenue au moyen d'un isolant dont la conductivité doit être faible de préférence inférieure à 0,1 w/m/k et dont la capacité calorifique doit également être très faible, de préférence inférieure à $2/10^5$ J/kg/k.

La mesure des températures permettant le calcul en particulier de la conductivité s'effectue au moyen de thermocouples intégrés à la plaque centrale 1 et sur les parois des plaques des échangeurs de chaleur 3a et/ou 3b. Grâce à cette implantation particulière des capteurs de températures, il n'est pas nécessaire d'implanter des capteurs internes dans l'échantillon de matériau à caractériser, ce qui permet une mise en oeuvre rapide et efficace de la manipulation sans détérioration de l'échantillon. Parmi ces capteurs de température, on distingue un premier capteur constitué par un thermocouple T2 intégré à la plaque centrale de séparation 1, et implanté de préférence au voisinage du centre de symétrie du conductivimètre constitué par la plaque centrale et les échangeurs de chaleur à plaques. Les deux autres thermocouples T1 et T3 permettant également des mesures de température sont intégrés sur la paroi des plaques des échangeurs. On préférera de préférence les intégrer au voisinage immédiat de la surface externe desdites plaques au plus près du centre de chacune des parois. Cette intégration est généralement réalisée au moyen de fines rainures ménagées à la surface desdites plaques et dans lesquelles viennent reposer les thermocouples. Grâce au fait que la plaque unique de séparation 1 est soumise à deux flux de chaleur sensiblement identiques provenant des deux échangeurs de chaleur, lesdits flux $\Phi a$, $\Phi b$ étant de direction opposée, on peut considérer que la plaque centrale de séparation 1 est isotherme lorsque l'équilibre thermique de l'ensemble du dispositif a été atteint. En raison de cette hypothèse portant sur la plaque centrale de séparation 1, on préférera utiliser une plaque centrale de séparation 1 à faces parallèles usinée de telle sorte que la tolérance sur l'épaisseur constante ne soit pas inférieure à 0,1 %. Pour confirmer l'hypothèse consistant à supposer que la température demeure uniforme pendant toute la durée de la mesure, il conviendra, d'une part que la plaque centrale soit réalisée en un matériau suffisamment bon conducteur de la chaleur, d'autre part que l'épaisseur Ep de la plaque centrale 1 soit choisie par rapport à l'épaisseur E de l'échantillon de telle sorte que les inégalités (A) et (B) suivantes soient respectées :

$$(A) \qquad (Ep/E)^2 < 0,1 \ \alpha P/\alpha E. \ 1/\Delta T$$

et

$$(B) \qquad Ep/E \leq \text{à } 0,5,$$

dans lesquelles
$\alpha_P$ et $\alpha_E$ sont les diffusivités respectives de la plaque et des échantillons et T est la plage de température imposée de l'extérieur.

L'échantillon ne peut pas quant à lui présenter des dimensions quelconques. En effet, pour obtenir une bonne précision de mesure, il est nécessaire que le rapport (épaisseur/rayon ou longueur de l'échantillon) soit plus petit que un tiers. Il est bien évident que cependant l'échantillon peut affecter n'importe quelle forme, circulaire, rectangulaire, etc sous réserve que l'isolant épouse parfaitement la périphérie dudit échantillon. L'épaisseur de l'échantillon du matériau à caractériser doit en outre répondre à l'in$\Delta$égalité suivante :

$$E^2 \leq \lambda \times T \times \pi^2/\gamma \ Cp,$$

dans laquelle $\lambda, \gamma$, Cp sont respectivement la conductivité, la masse volumique et la chaleur spécifique volumique dans l'intervalle de température considéré de l'échantillon du matériau à caractériser. T constitue le délai nécessaire pour obtenir la mise en équilibre thermique de l'ensemble du dispositif, cet équilibre thermique étant nécessaire pour considérer la plaque comme un accumulateur de chaleur dont à tout moment la température est homogène. En conséquence, pour permettre une mesure rapide de l'échantillon, il faudra fixer cette période T à un temps quelconque mais relativement raisonnable de l'ordre de la demi-heure ou de l'heure. Il suffira donc pour obtenir cette mise en oeuvre rapide de choisir une épaisseur E d'échantillon faible ce qui constitue une mesure peu contraignante.

Dans ces conditions, la plaque centrale de séparation 1 étant à tout moment à une température homogène, il est possible de connaître la valeur des flux échangés entre les échantillons 2a, 2b et la plaque de séparation 1.

Ainsi, dans les conditions énoncées ci-dessus, un transfert global de chaleur H pendant un intervalle de temps t provoque une élévation de température $\Delta$ T : H = m Cp $\Delta$ T.

Si S est la surface latérale de la plaque, $\gamma$, Cp les valeurs caractéristiques de son matériau, on peut directement calculer le flux de chaleur moyen entre t et t + $\Delta$ t :

$$\Phi m = \Delta H/2 S \times \Delta t = 1/2 \ \gamma \ Cp \ E \ p \ \Delta T/\Delta t$$

La mesure de la masse volumique $\gamma$ ne pose généralement pas de problème et peut être réalisée avec une bonne précision. Quant à la tolérance sur E, p elle est faible par construction. La connaissance de ces flux permet par la suite une identification précise des conductivités et des valeurs de résistance de contact. Un exemple de réalisation d'un conductivimètre et de la détermination de la conductivité d'un échantillon d'un matériau à caractériser seront fournis ci-après.

Les données obtenues sont constituées notamment par les courbes de températures T1 (t), T2 (t), T3 (t). Pour identifier la conductivité de l'échantillon du matériau à caractériser, on utilise une méthode d'identification dont le schéma fonctionnel est représenté à la figure 2. Dans cette méthode d'identification, on considère un système physique constitué par le conductivimètre proprement dit tel que décrit ci-dessus. Ce système physique est soumis à une excitation connue permettant l'obtention d'une évolution de la température par rapport au temps, cette évolution est appelée la réponse objet (T1 (t), T2 (t), T3 (t)). Parallèlement à ce système physique, on considère un modèle constitué par un système thermique qui est décrit par une série d'équations différentielles partielles non linéaires dans une plage de temps [O,tF] et dans un espace [0, L], L correspondant à la distance séparant une plaque d'échangeur de chaleur de la plaque de séparation centrale. Ce système permet d'obtenir un certain nombre de solutions calculées à partir des points de mesure obtenus à partir du système physique. Ces solutions sont appelées réponse du modèle. Cette réponse dépend de P paramètres inconnus de l'équation. Ces paramètres forment le vecteur $\beta$ $\varepsilon$ qui doit être identifié. est appelé "série de paramètres admissibles" et en pratique, la série est définie par leurs valeurs minimale et maximale. Dans le problème traité ici, $\beta$ est défini par

$$(1) \qquad \beta = (\lambda l,...,\lambda i,...,\lambda_{NI}, R1_{l},...,R1_{j},...,R1_{NJ},...,$$

$$R2_{l},...,R2_{l},...,R2_{NL})$$

$$(1a) \qquad p = NI + NJ + NL$$

où $\lambda$ i est la conductivité pour les températures $\Theta$i et $R1_{j}$ et $R2_{l}$, les résistances thermiques de contact pour les instants j et l respectivement.

L'identification consiste en la détermination de $\beta$ *, une valeur de $\beta$, afin que la réponse du modèle soit aussi proche que possible, le critère restant à définir, de la réponse de l'objet. Pour trouver $\beta$ *, une méthode de descente est utilisée. C'est une méthode itérative qui consiste à calculer une suite $\beta 0$, $\beta 1$, $\beta l$ (dans $\wedge$ ) qui satisfasse $J(\beta 0) > J(\beta 1) > ... > J\beta i$.

La température de l'échantillon $\Theta$ (x, t) dans le domaine spatial (O, L) limité par les deux interfaces et la température $\Theta_1$ de la première couche à l'interface x = 0, sont une solution de :

$$C(\theta) \frac{\partial \theta}{\partial t}(x,t) - \frac{\partial}{\partial x}\left[\lambda(\theta)\frac{\partial \theta}{\partial x}(x,t)\right] = 0 \qquad\qquad 0 < x < L \qquad\qquad (3)$$

$$\theta_1(t) = \theta(0,t) - R1(t)\phi_1(t) \qquad x = 0 \qquad\qquad (3a)$$

$$-\lambda(\theta)\frac{\partial \theta}{\partial x}(0,t) = \phi_1(t) \qquad x = 0 \qquad\qquad (3b)$$

$$-R2(t)\left[\lambda(\theta)\frac{\partial \theta}{\partial x}(L,t)\right] + \theta(L,t) = T_2(t) \qquad\qquad x = L \qquad (3c)$$

Les données sont :

- mesures : $\Phi_1(t)$, $T_1(t)$ et $T_2(t)$, pour $0 < t < t_f$
- paramètres : $C(\Theta) = (\Theta) Cp (\Theta)$, et L
- conditions initiales : $\Theta_1$ (0) et $\Theta$ (x, 0) pour $0 < x < L$

L'expression général du critère J est :

$$J(\beta) = \sum_{k=1}^{n} ( \theta_1(t_k, \beta) - T_1(t_k) )^2 w_k + \sum_{i=1}^{p} (\mu_i - \beta_i)^2 u_i \qquad (4)$$

où :

$w_k$ - est un coefficient de poids positif pour l'instant k, qui

peut inclure quelques informations selon les erreurs expérimentales,

$u_i$ - est un coefficient de poids non négatif pour i-th composant de $\beta$,

$\mu_i$ - est une estimation initiale de $\beta$.

Ce critère d'écart J ($\beta$) permet de minimiser l'écart entre les températures mesurées dans la plaque centrale et les températures calculées pour le même point et dans les mêmes instants par le modèle. Cet ajustement est fait par une méthode inverse basée sur le principe de méthode de Gauss-Newton (Voir J.V. Beck "Parameter estimation in Engineering and Science", Wiley 1977).

Cette minimisation a un caractère itératif, c'est-à-dire que les caractères sont successivement ajustés jusqu'à ce que le critère d'écart J ($\beta$) soit égal à une valeur qui correspond à une condition d'arrêt du calcul.

Ainsi, à titre d'exemple, supposons un dispositif conforme à l'invention du type ci-dessous.

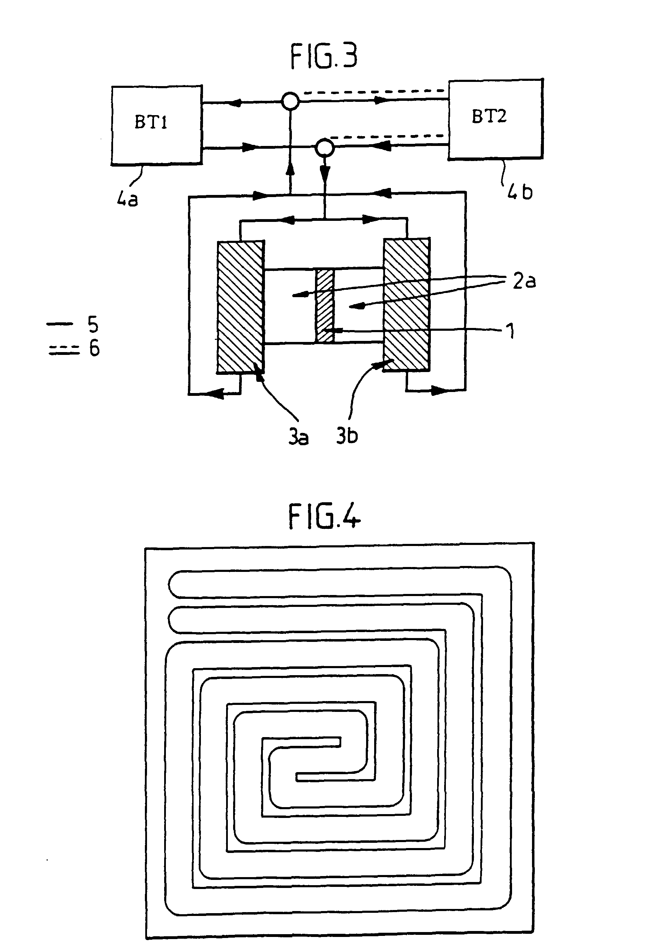

Les échangeurs latéraux 3a et 3b sont des échangeurs classiques en soi-connus du type à circulation de fluide caloporteur comme le montre la figure 4. Ce fluide provient d'un bain thermostaté (4a ou 4b). Le débit du fluide à l'intérieur de l'échangeur est généralement de l'ordre de 20 1/mn. Ces échangeurs sont généralement en aluminium.

La plaque centrale 1 est également en aluminium et présente par exemple les dimensions suivantes : 140 x 140 x 4 mm.

Quant aux échantillons, ils se présentent dans l'exemple considéré sous forme de plaques de plexiglass (140 x 140 x 12 mm).

De ce fait : $(Ep/E)^2 < 0{,}1$ $\alpha p/\alpha E$ . $1/\Delta T$ correspond à $(4/12)^2 < 0{,}1$ $\underline{8{,}42 \times 10^{-5}}$ . 1

L'isolant est quant à lui constitué par du polystyrène.

Les échangeurs de chaleur sont alimentés au moyen de deux circuits de fluides 5 et 6 représentés à la figure 3. Dans un premier temps, au départ de la manipulation, seul le circuit 5 est utilisé. Ce circuit fonctionne pendant plusieurs minutes et permet l'obtention d'une température initiale T0 de la plaque centrale 1 correspondant à l'une des valeurs extrêmes de la plage de température étudiée. Lorsque cette température est constante, et donc que le circuit est en équilibre thermique, on peut procéder à la mesure. A l'instant t = 0 de la mesure, les circuits 5 et 6 sont inversés et c'est alors le circuit 6 qui va assurer le chauffage ou le refroidissement des échantillons. On utilise deux bains thermostatés pour diminuer la durée de la manipulation.

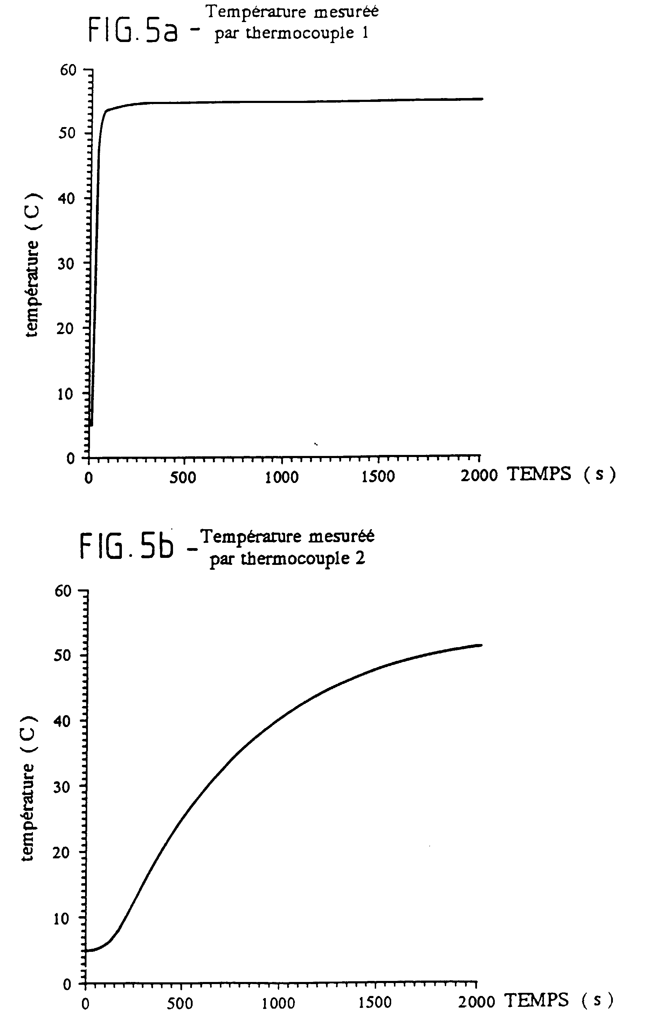

On obtient ainsi les courbes T = f (t) représentées aux figures 5a et 5b. Bien évidemment la courbe susceptible d'être obtenue à partir du thermocouple T3 n'a pas été représentée car elle aurait été sauf incident technique identique à celle de la figure 5a.

Cette acquisition des données est réalisée au moyen d'un ordinateur type PC et d'un voltmètre par exemple (scanner Keithley 199 fabriqué par Keithley).

Grâce aux données obtenues par l'intermédiaire du thermocouple T2 de la plaque centrale 1, on peut calculer le flux de chaleur faisant varier la température de la plaque centrale et traversant une face de l'échantillon en appliquant la formule suivante :

$$\Phi p = 1/2\ \gamma\ Cp\ E\ p\ \Delta T/ \Delta t$$

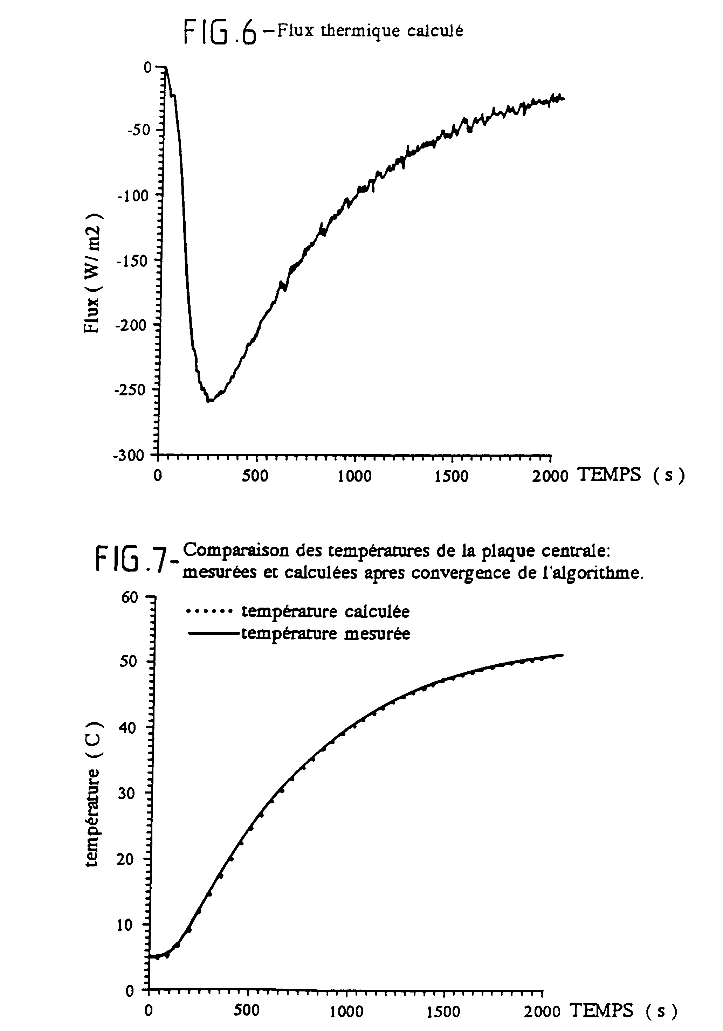

On obtient alors une courbe $\Phi p = f (t)$ conforme à celle représentée à la figure 6.

Puis dans une troisième étape, on initialise les paramètres identifiés : dans l'exemple considéré, les paramètres identifiés sont :

la conductivité thermique du plexiglass (échantillon) $\lambda$ et les résistances de contact aluminium/plexiglass : R1, R2 et ont pour valeur

$\lambda = 0{,}189\ Wm^{-1}\ k^{-1}$

$R1 = R2 = 1.10^{-5}\ km^2\ W^{-1}$

On introduit les données suivantes :

- chaleur spécifique volumique de l'échantillon :

$$Cp = 10527.10^6 \ Jk^{-1} \ m^{-2}$$

- épaisseur de l'échantillon.

On constate qu'une erreur importante peut être commise sur la valeur de Cp sans affecter de manière significative la valeur de la conductivité identifiée de l'échantillon.

On applique à ces données l'algorithme d'identification en prenant comme conditions limites le flux, L et la température correspondant respectivement aux figures 6 et 5a. La température de plaque est ensuite calculée. On résout l'équation de chaleur de manière à obtenir un ensemble de résultats de calcul puis on calcule le critère J décrit précédemment.

Si ce critère J répond à la condition d'arrêt, la valeur de la conductivité est optimale sinon on modifie les paramètres optimisés au moyen de la méthode de Gauss Newton.

On montre sur la figure 7 la comparaison entre la température de plaque calculée et cette température mesurée, lorsque l'algorithme a convergé.

On constate que les valeurs identifiées pour les résistances sont très faibles de l'ordre de $1.10^{-5}$ car le contact était presque parfait. La valeur identifiée pour la conductivité est constante et très proche des valeurs de référence ($\lambda = 0,19 \ Wm^{-1} \ k^{-1}$).

Le dispositif de mesure et la méthode d'identification associée peuvent être utilisés par les fabricants de logiciels, les fabricants de matières en vue de tester les caractéristiques de leurs produits, les transformateurs de matières notamment dans le cadre de procédures de contrôle qualité.

## Revendications

1. Dispositif de mesure pour mesurer en régime transitoire la conductivité thermique et/ou la capacité calorifique d'un matériau injectable ou non comprenant deux échangeurs de chaleur (3a, 3b) à paroi plane disposés de part et d'autre d'une plaque plane (1) à parois parallèles suffisamment conductrice de la chaleur pour être supposée isotherme et délimitant avec ladite plaque deux chambres sensiblement identiques, des moyens de chauffage et/ou de refroidissement (5, 6) desdits échangeurs de chaleur (3a, 3b), deux échantillons (2a, 2b) du matériau à caractériser d'épaisseur similaire (E), disposés dans lesdites chambres respectivement entre la face externe de l'échangeur (3a, 3b) et une face de la plaque centrale (1) de séparation de manière à être traversés par un flux de chaleur ($\phi a$, $\phi b$), un dispositif de positionnement et de maintien des échangeurs (3a, 3b) sensiblement parallèles à la plaque centrale (1) de manière à assurer un contact de pression homogène entre les parois des échangeurs (3a, 3b), les faces externes des échantillons (2a, 2b) et les faces de la plaque centrale (1), des moyens d'isolation fermant les bords restant libres desdites chambres pour assurer un flux de chaleur ($\phi a$, $\phi b$) unidirectionnel, et au moins deux capteurs de température dont l'un (T2), constitué par un thermocouple, est intégré à la plaque centrale (1) et dont l'autre (TI), constitué également par un thermocouple, est intégré à la paroi externe de la plaque d'un des échangeurs (3a).

2. Dispositif de mesure selon la revendication 1,
caractérisé en ce qu'il comprend un troisième capteur de température constitué par un thermocouple (T3) intégré sur la paroi externe de la plaque du deuxième échangeur (3b).

3. Dispositif de mesure selon l'une des revendications 1 et 2,
caractérisé en ce que les épaisseurs respectives (Ep et E) de la plaque centrale de séparation (1) et de l'échantillon (2a ou 2b) à caractériser sont choisies de manière telles que :

$$(Ep/E^2) < 0,1 \ \alpha P/\alpha E. \ 1/\Delta \ \theta$$

et

$$Ep/E \leq à \ 0,5,$$

$\alpha P$ et $\alpha E$ étant les diffusivités respectives de la plaque (1) et de l'échantillon (2a, 2b), $\Delta \Theta$ constituant la plage de variation de la température imposée par lesdits moyens de chauffage et/ou de refroidissement (5, 6).

**4.** Dispositif de mesure selon l'une des revendications 1 à 3,
caractérisé en ce que l'isolant présente une conductivité thermique inférieure à 0,1 w/m/k et une capacité calorifique inférieure à $2.10^5$ J/kg/K.

**5.** Dispositif de mesure selon l'une des revendications 1 à 4,
caractérisé en ce que l'enregistrement des courbes de températures T1 (t), T2 (t), T3 (t) des thermocouples est réalisé sur une période [t, t+ $\Delta$ t] pour une variation de température [$\Theta$, $\Theta$ + $\Delta$ $\Theta$] uniquement lorsque l'équilibre thermique du dispositif est atteint, c'est-à-dire lorsque l'évolution de T1 (t) est identique à celle de T2 (t).

**6.** Dispositif de mesure selon l'une des revendications 1 à 5,
caractérisé en ce que l'épaisseur E de l'échantillon (2a, 2b) à caractériser satisfait la relation suivante

$$E2 \leq \lambda \, T \, \pi^2 \, / \, \gamma \, Cp.$$

$\lambda$, $\gamma$, Cp constituant respectivement la conductivité, la masse volumique, la chaleur spécifique volumique dans le domaine de température considérée de l'échantillon du matériau à caractériser, de telle sorte que la période T est la période maximale nécessaire et suffisante avant de pouvoir réaliser les mesures.

**7.** Dispositif de mesure selon la revendication 1,
caractérisé en ce que le dispositif de positionnement et de maintien des échangeurs est constitué par les plateaux mobiles d'une presse dont les faces en regard sont solidarisées auxdits échangeurs.

**8.** Procédé d'identification de la conductivité optimale d'un matériau au moyen d'un dispositif de mesure conforme à la revendication 1, fournissant au moins les courbes (T1 (t), T2 (t)) appelées par la suite réponse objet,
caractérisé en ce qu'on réalise un modèle du type différences finies permettant d'obtenir une réponse modèle $\Theta$ (t), en ce qu'on définit un critère d'écart

$$J = \sum_{k=1}^{n} (\Theta_k - T_k)^2$$

$\Theta_k$ : température calculée à l'instant k
$T_k$ : température mesurée à l'instant k

qui permet de minimiser l'écart entre les températures mesurées dans la plaque centrale et les températures calculées pour le même point et dans les mêmes instants pour le modèle, ladite minimisation présentant un caractère itératif de manière à ajuster successivement l'ensemble des paramètres selon une méthode de descente, en ce qu'on vérifie à chaque nouvelle valeur du critère si elle satisfait à la condition d'arrêt constituée par l'égalité $J = \Sigma$, $\Sigma$ étant considéré comme négligeable de manière à soit, arrêter le calcul, la valeur optimale de la conductivité ayant été obtenue soit à recommencer les calculs cités précédemment.

**Patentansprüche**

**1.** Meßvorrichtung zum Messen der Wärmeleitfähigkeit und/oder der Wärmekapazität eines spritzbaren oder nicht spritzbaren Materials im vorübergehenden Zustand, mit zwei Wärmetauschern (3a, 3b) mit ebener Wandung, die auf beiden Seiten einer ebenen Platte mit parallelen Wandungen, die ausreichend wärmeleitend ist, um als isotherm angenommen zu werden, angeordnet sind und mit der Platte zwei im wesentlichen identische Kammern begrenzen, mit Mitteln (5, 6) zum Erwärmen und/oder Abkühlen der Wärmetauscher (3a, 3b), mit zwei Proben (2a, 2b) des zu charakterisierenden Materials, die von ähnlicher Dicke (E) sind und die in den Kammern jeweils zwischen der Außenfläche des Tauschers (3a, 3b) und einer Fläche der zentralen Trennplatte (1) angeordnet sind, um von einem Wärmefluß ($\phi$a,$\phi$b) durchquert zu werden, mit einer Vorrichtung zur Positionierung und zum Halten der Tauscher (3a, 3b) im wesentlichen parallel zu der zentralen Platte (1), um einen homogenen Druckkontakt zwischen den Wandungen der Tauscher (3a, 3b), den Außenflächen der Proben (2a, 2b) und den Flächen der zentralen Platte (1) sicherzustellen, mit Isolationsmitteln, die die verbleibenden freien Ränder der Kammern verschließen, um einen undidirektionalen Wärmefluß ($\phi$a,$\phi$b) sicherzustellen, und mit wenigstens zwei Temperaturaufnehmern, von denen der eine (T2), der durch ein Thermoelement gebildet ist, in die zentrale Platte (1) integriert ist und von denen der andere (T1), der ebenfalls durch ein Thermoelement gebildet ist, in die äußere Wandung der Platte eines der Tauscher (3a) integriert ist.

**2.** Meßvorrichtung nach Anspruch 1,
dadurch gekennzeichnet, daß sie einen dritten Temperaturaufnehmer aufweist, der durch ein Thermoelement (T3) gebildet und in die äußere Wandung der Platte des zweiten Tauschers (3b) integriert ist.

**3.** Meßvorrichtung nach Anspruch 1 oder 2,
dadurch gekennzeichnet, daß die jeweiligen Dicken (Ep und E) der zentralen Trennplatte (1) und der zu charakterisierenden Probe (2a oder 2b) derart gewählt sind, daß:

$$(Ep/E^2) < 0{,}1 \ \alpha P/\alpha E. \ 1/\Delta \ \Theta$$

und

$$Ep/E \leqslant 7 \ 0{,}5,$$

wobei $\alpha P$ und $\alpha E$ die Wärmeleitzahlen der Platte (1) bzw. der Probe (2a, 2b) sind, wobei $\Delta \ \Theta$ den Variationsbereich der Temperatur bildet, der durch die Mittel (5, 6) zum Erwärmen und/oder zum Abkühlen aufgeprägt wird.

**4.** Meßvorrichtung nach einem der Ansprüche 1 bis 3,
dadurch gekennzeichnet, daß das Isoliermaterial eine Wärmeleitfähigkeit, die geringer ist als 0,1 w/m/k, und eine Wärmekapazität, die geringer ist als $2.10^5$J/kg/K, aufweist.

**5.** Meßvorrichtung nach einem der Ansprüche 1 bis 4,
dadurch gekennzeichnet, daß die Aufzeichnung von Temperaturkurven T1(t), T2(t) und T3(t) der Thermoelemente über einen Zeitraum [t, t+$\Delta$t] für eine Temperaturvariation [$\Theta$,$\Theta$+$\Delta\Theta$] nur dann vorgenommen wird, wenn das thermische Gleichgewicht der Vorrichtung erreicht ist, d.h. wenn die Entwicklung der Temperatur T1(t) identisch zu derjenigen der Temperatur T2(t) ist.

**6.** Meßvorrichtung nach einem der Ansprüche 1 bis 5,
dadurch gekennzeichnet, daß die Dicke E der zu charakterisierenden Probe (3a, 3b) der folgenden Gleichung genügt

$$E^2 \leqslant \lambda \ T \ \pi^2 / \gamma \ Cp$$

wobei $\lambda$, $\gamma$, Cp die Leitfähigkeit bzw. die Dichte bzw. die molare spezifische Wärme der Probe des zu charakterisierenden Materials in dem betrachteten Temperaturbereich darstellt, derart, daß der Zeitraum T der maximal erforderliche und ausreichende Zeitraum ist, bevor die Messungen ausgeführt werden können.

**7.** Meßvorrichtung nach Anspruch 1,
dadurch gekennzeichnet, daß die Vorrichtung zum Positionieren und zum Halten der Tauscher durch die beweglichen Platten einer Presse gebildet ist, deren gegenüberliegende Flächen mit den Tauschern verbunden sind.

**8.** Verfahren zur Identifizierung der optimalen Leitfähigkeit eines Materiales mittels einer Meßvorrichtung gemäß Anspruch 1, das wenigstens die Kurven (T1(t), T2(t)) liefert, die in der Folge als Objektantwort bezeichnet werden, dadurch gekennzeichnet, daß ein Modell nach Art der abgeschlossenen Differenzen realisiert wird, das es ermöglicht, eine Modellantwort $\Theta$(t) zu erhalten, daß ein Abstandskriterium definiert wird

$$J = \sum_{k=1}^{n} (\Theta_k - T_k)^2$$

$\Theta_k$ : berechnete Temperatur zum Zeitpunkt k

$T_k$ : gemessene Temperatur zum Zeitpunkt k

das es ermöglicht, den Abstand zwischen den in der zentralen Platte gemessenen Temperaturen und den berechneten Temperaturen für das Modell für denselben Punkt und in denselben Zeitpunkten zu minimieren, wobei die Minimierung einen iterativen Charakter aufweist, um sukzessive die Gesamtheit der Parameter nach einer absteigenden Methode anzupassen,

daß bei jedem neuen Wert des Kriteriums überprüft wird, ob er dem Endzustand genügt, der durch die Gleichung J=Σ gebildet wird, wobei Σ als vernachlässigbar betrachtet wird, um entweder die Berechnung zu beenden, wobei der optimale Wert der Leitfähigkeit erzielt ist, oder wieder die vorgenannten Berechnungen zu beginnen.

## Claims

1. A measuring device for measuring the thermal conductivity and/or the calorific capacity in a transitory regime of an injectable or non-injectable material, comprising two plane wall heat exchangers (3a, 3b) located on one side and the other of a flat plate (1) with parallel walls, sufficiently conductive of heat to be regarded as isothermal and bounding with the said plate two substantially identical chambers, means for heating and/or cooling (5, 6) the said heat exchangers (3a, 3b), two specimens (2a, 2b) of the material to be characterized, of similar thickness (E), located in the said chambers respectively between the external face of the exchanger (3a, 3b) and on face of the central separating plate (1), in such a manner as to be traversed by a heat flux ($\phi$a, $\phi$b), a device for positioning and maintaining the exchangers (3a, 3b) substantially parallel to the central plate (1) in such a manner as to ensure contact under uniform pressure between the walls of the exchangers (3a, 3b), the external faces of the specimens (2a, 2b) and the faces of the central plate (1), insulating means closing the edges remaining free of the said chambers in order to ensure a unidirectional heat flux ($\phi$a, $\phi$b), and at least two temperature sensors of which one (T2), formed by a thermocouple, is integrated in the central plate (1) and of which the other (T1), also formed by a thermocouple, is integrated in the external wall of the plate of one of the exchangers (3a).

2. A measuring device according to claim 1, characterized in that it comprises a third temperature sensor formed by a thermocouple (T3) integrated in the external wall of the plate of the second exchanger (3b).

3. A measuring device according to claim 1 or 2, characterized in that the respective thicknesses (Ep and E) of the central separating plate (1) and the specimen (2a or 2b) to be characterized are selected in a manner such that:

$$(Ep/E^2) < 0.1 \ \alpha P/\alpha E, \ 1/\Delta\Theta$$

and

$$Ep/E \leq 0.5,$$

$\alpha$P and $\alpha$E being the respective diffusivities of the plate (1) and the specimen (2a, 2b), $\Delta\Theta$ being the range of variation of temperature imposed by the said heating and/or cooling means (5, 6).

4. A measuring device according to any of claims 1 to 3, characterized in that the insulation has a thermal conductivity lower than 0.1 w/m/K and a thermal capacity lower than $2.10^5$ J/kg/K.

5. A measuring device according to any of claims 1 to 4, characterized in that recording of temperature curves T1(t), T2(t), T3(t) of the thermocouples is effected over a period [t, t+$\Delta$t] for a variation in temperature [$\Theta$, $\Theta$+$\Delta\Theta$] only when thermal equilibrium of the device has been attained, i.e. when the change of T1(t) is identical to that of T2(t).

6. A measuring device according to any of claims 1 to 5, characterized in that the thickness E of the specimen (2a, 2b) to be characterized satisfies the following relation:

$$E^2 \leq \lambda T \pi^2 / \gamma\, Cp$$

$\lambda$, $\gamma$, Cp being respectively the conductivity, the specific gravity and the volumetric specific heat in the temperature range involved of the specimen of the material to be characterized, in such a manner that the time T is the maximum time necessary and sufficient before being able to effect the measurement.

7. A measuring device according to claim 1, characterized in that the device for positioning and maintaining the exchangers is formed by movable plates of a press whose confronting faces are fixed to the said exchangers.

8. A method of identifying the optimum conductivity of a material by means of a measuring device in accordance with claim 1, providing at least the curves (T1(t), T2(t)) provided by the succession of object responses, characterized in that a model of finite difference type is created allowing a model response $\Theta(t)$ to be obtained, in that a divergence criterion is defined

$$J = \sum_{k=1}^{n} (\theta_k - T_k)^2$$

where

$\Theta_k$ is the calculated temperature at the instant k
and $T_k$ is the measured temperature at the instant k

which enables the divergence between the temperatures measured in the central plate and the calculated temperatures for the same point and at the same instants for the model, the said minimisation taking place iteratively in such a manner as to adjust successively the set of parameters according to a method of successive approximation, and in that a check is made for each new value of the criterion whether it satisfies the stop condition given by the equality $J = \Sigma$, $\Sigma$ being taken to be negligible in such a manner as either to stop the calculation, the optimum value of the conductivity having been obtained, or to recommence the calculations recited above.

# FIG.1

# FIG.2

# FIG.3

# FIG.4

FIG.5a – Température mesurée
par thermocouple 1

FIG.5b – Température mesurée
par thermocouple 2

14

FIG.6 – Flux thermique calculé

FIG.7 – Comparaison des températures de la plaque centrale: mesurées et calculées apres convergence de l'algorithme.

# FIG . 8